# FASCICULE DE BREVET EUROPEEN

(11) **EP 0 959 771 B1**
(45) Date de publication et mention de la délivrance du brevet: **08.10.2003**
(21) Numéro de dépôt: 97932857.2
(22) Date de dépôt: 08.07.1997
(51) Int. Cl.: A61B 6/00, G03B 42/04

(54) **DISPOSITIF MOBILE DE RADIOGRAPHIE AVEC CONTENEUR DE CASSETTES**
FAHRBARES RÖNTGENGERÄT MIT KASSETTENBEHÄLTER
MOBILE X-RAY DEVICE WITH CASSETTE CONTAINER

(30) Priorité: 09.07.1996 FR 9608543
(43) Date de publication de la demande: 01.12.1999
(73) Titulaire: GE MEDICAL SYSTEMS SA, 78533 Buc Cedex (FR)
(72) Inventeur: MORASSE, Louis, F-78533 Buc Cedex (FR)
(74) Mandataire: Goode, Ian Roy
(86) Numéro de dépôt international: FR9701233
(87) Numéro de publication internationale: WO98001072

(56) Documents cités:
- EP-A- 0 000 691
- DE-A- 3 138 916
- DE-C- 872 730

## Description

La présente invention concerne un dispositif mobile de radiographie du genre utilisé dans les hôpitaux, pour effectuer des prises d'images radiologiques de différentes parties du corps d'un patient, dans la chambre de celui-ci, afin d'éviter d'avoir à le déplacer.

On amène donc le dispositif mobile de radiographie à proximité du lit du patient et on le dispose de façon à prendre des clichés de la partie du corps du patient qui doit être soumise à un diagnostic.

Les dispositifs mobiles de radiographie comprennent en général un châssis à roues, un générateur de rayons X et un bac pour plusieurs cassettes. Ces cassettes contiennent chacune un film impressionnable par les rayons X. Le dispositif mobile de radiographie effectue donc un parcours dans les chambres des différents patients au cours duquel les cassettes sont utilisées une par une. Une cassette vierge est prise dans le bac avant la prise du cliché, puis la cassette dont le film est impressionné est remise dans le bac après la prise du cliché. Les cassettes dont le film est impressionné sont ensuite transportées manuellement par un opérateur jusqu'à une chambre de traitement située en général dans l'hôpital.

Toutefois, les cassettes pèsent aux alentours de 900 g chacune et l'opérateur chargé de leur transport jusqu'à la chambre de traitement doit couramment en porter une dizaine. Une telle charge est lourde à transporter et, les cassettes étant en forme de parallélépipède plat, il est difficile d'en prendre une dizaine à la fois sans risquer de les faire tomber. Une chute éventuelle risquerait de détériorer les cassettes qui sont relativement onéreuses et d'obliger à refaire les clichés.

La présente invention a donc pour objet de remédier aux inconvénients précités.

La présente invention a pour objet de proposer un dispositif mobile de radiographie dans lequel tout risque de détérioration des cassettes par chute est évité et dans lequel la pénibilité du travail des opérateurs est réduite.

Le dispositif mobile de radiographie, selon l'invention, est du type comprenant un châssis à roues, un générateur de rayons X et un conteneur, pour une pluralité de cassettes contenant chacune un film impressionnable par les rayons X et destiné à être développé dans une chambre de traitement.

Le conteneur de cassettes est séparable du dispositif mobile de radiographie pour le transport desdites cassettes vers la chambre de traitement, le conteneur comprenant des moyens de roulement et des moyens de préhension par un opérateur.

L'opérateur peut ainsi emmener un lot de cassettes dans la chambre de traitement sans avoir à les porter.

Dans un mode de réalisation de l'invention, le dispositif mobile de radiographie comprend, sur une surface verticale, un logement capable de recevoir le conteneur, ledit logement étant pourvu de moyens pour maintenir le conteneur dans une position fermée, de moyens pour maintenir le conteneur dans une position ouverte et de moyens pour pivoter le conteneur entre lesdites positions fermée et ouverte et permettant la séparation du contenur du dispositif mobile de radiographie.

Avantageusement, le conteneur comprend une paire de galets de roulement ou d'éléments de support en contact avec le sol lorsque le conteneur est séparé du dispositif mobile de radiographie et surélevés par rapport au sol lorsque le conteneur est inséré dans le logement. Le conteneur peut comprendre une béquille de contact avec le sol formant un triangle avec les galets de roulement ou les éléments de support pour la stabilité du conteneur, ledit conteneur étant séparé du dispositif mobile de radiographie.

Avantageusement, le conteneur peut comprendre des moyens pour se surélever par rapport au sol lors de l'insertion dans le logement. On évite ainsi un accroissement de la résistance au roulement du dispositif mobile de radiographie.

Dans un mode de réalisation de l'invention, le dispositif mobile de radiographie comprend un axe formé par deux doigts faisant saillie l'un en face de l'autre dans une portion inférieure du logement, et capables de coopérer avec des creux correspondants du conteneur pour le pivotement dudit conteneur autour desdits doigts.

Dans un mode de réalisation de l'invention, le dispositif mobile de radiographie comprend des moyens élastiques de maintien du conteneur en position fermée, disposés dans une partie supérieure du logement.

Dans un mode de réalisation préféré de l'invention, le logement comprend au moins une rampe coopérant avec au moins une protubérance du conteneur, la protubérance du conteneur étant disposée à une extrémité de la rampe lorsque ledit conteneur est en position fermée, ladite protubérance étant en contact avec un bossage de maintien disposé sur la rampe lorsque le conteneur est en position ouverte et ladite protubérance étant capable de franchir le bossage de maintien en vue de la séparation du conteneur et du dispositif mobile de radiographie.

Dans un mode de réalisation de l'invention, le conteneur comprend une poignée de transport. Cette poignée peut être rétractable pour être insérée dans le logement et éviter de former une aspérité gênant l'utilisation du dispositif mobile de radiographie.

Grâce à l'invention, on obtient un conteneur de cassettes, amovible par rapport au dispositif de radiographie et pouvant être disposé dans une position fermée, dans une position ouverte, par exemple pour y prendre ou y remettre une cassette et dans une position séparée du dispositif mobile de radiographie pour le transport des cassettes vers une chambre de traitement.

L'invention sera mieux comprise à l'étude de la description détaillée d'un mode de réalisation pris à titre d'exemple nullement limitatif et illustré par les dessins annexés, sur lesquels :
la figure 1 est une vue en perspective du dispositif mobile de radiographie et du conteneur amovible séparés l'un de l'autre;
la figure 2 est une vue en perspective du dispositif mobile de radiographie équipé du conteneur en position ouverte;
la figure 3 est une vue semblable à la figure 2 avec le conteneur en position fermée; et
la figure 4 est une vue schématique du profil de la rampe.

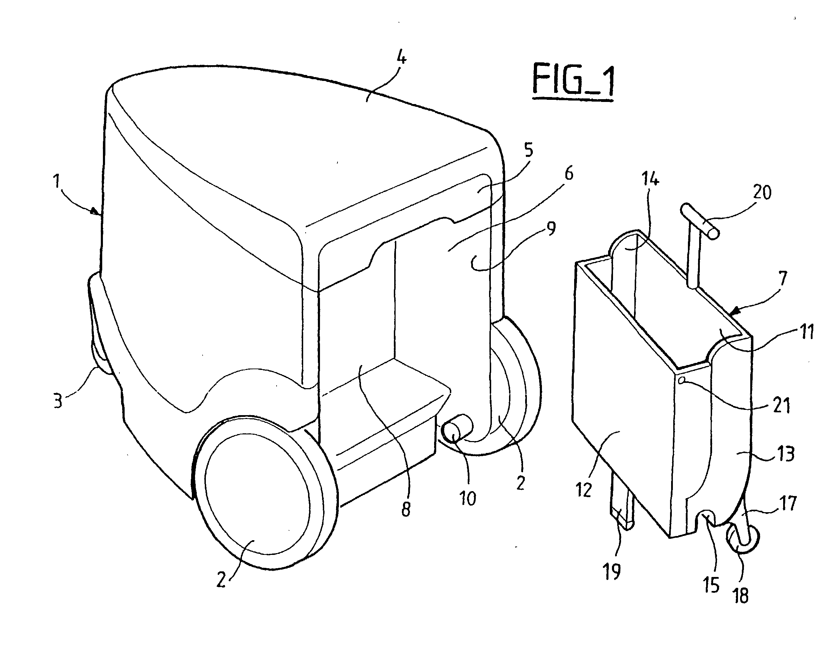

Comme on peut le voir sur les figures, l'appareil mobile de radiographie comprend un châssis 1 reposant sur le sol au moyen d'une paire de roues arrière 2 d'axe fixe et d'une roue avant 3 pivotante. Le châssis 1 comprend une face supérieure 4 et une face arrière 5. Sur la face supérieure 4, peuvent être prévus, un clavier, non représenté, de commande des opérations de radiographie, et un bras articulé, non représenté, supportant le générateur de rayons X et destiné à venir à proximité du corps du patient. La face arrière 5 est creuse de façon à définir un logement 6 destiné à recevoir le conteneur mobile 7. Le logement 6, de forme sensiblement parallélépipédique, comprend une paroi de fond 8 et deux parois latérales 9 dont une seule est visible sur la figure 1. L'extrémité inférieure de chaque paroi latérale 9 du logement 6 est pourvue d'un doigt 10, cylindrique, de diamètre correspondant sensiblement à sa longueur, perpendiculaire à la paroi 9 et faisant saillie vers l'intérieur du logement 6. Les deux doigts 10 sont coaxiaux et forment un axe de pivotement.

Le conteneur 7, de forme concordante à celle du logement 6, comprend une face supérieure creuse 11 destinée à recevoir les cassettes utilisées dans les appareils mobiles de radiographie et contenant en général un film impressionnable par les rayons X, le film impressionné étant ensuite soumis à une opération de développement.

Le conteneur 7 comprend également une face arrière 12 et deux faces latérales 13 et 14. La face latérale 13 comprend à son extrémité inférieure un creux 15 capable de coopérer avec un doigt 10 du logement 6. La face latérale 14 comprend également un creux identique au creux 15. Le conteneur 7 comprend une face avant 16 à proximité de l'extrémité inférieure de laquelle sont disposés deux pieds 17 supportant chacun un galet de roulement 18. Les pieds 17 se prolongent plus bas que l'extrémité inférieure de la face avant 16 de manière à être en contact avec le sol lorsque le conteneur 7 est séparé du dispositif mobile de radiographie (figure 1).

A l'opposé, du côté de la face arrière 12, le conteneur 7 comprend une béquille 19 capable d'entrer en contact avec le sol pour former avec les galets 18 un triangle de sustentation. Le conteneur 7 comprend, également, une poignée 20 qui fait saillie vers le haut à partir de la face supérieure 14. La poignée 20 est fixe, mais on pourrait prévoir une poignée rétractable pour éviter qu'elle ne soit accrochée par un opérateur accidentellement.

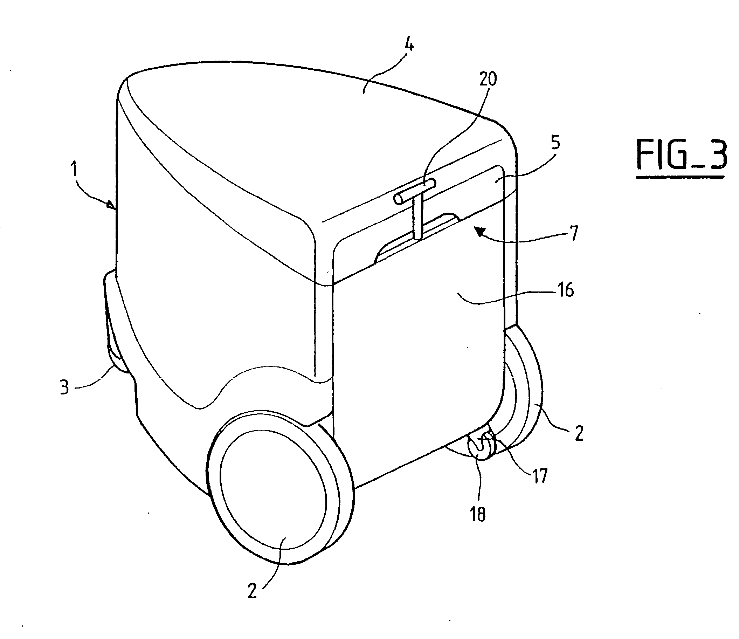

Lors de la prise d'un cliché, l'appareil de radiographie est dans la position illustrée sur la figure 3 avec le conteneur 7 en place dans son logement 6 et fermé. Les galets de roulement 18 et la béquille 19 sont alors surélevés par rapport au sol pour éviter d'accroître inutilement la résistance à l'avancement de l'appareil de radiographie.

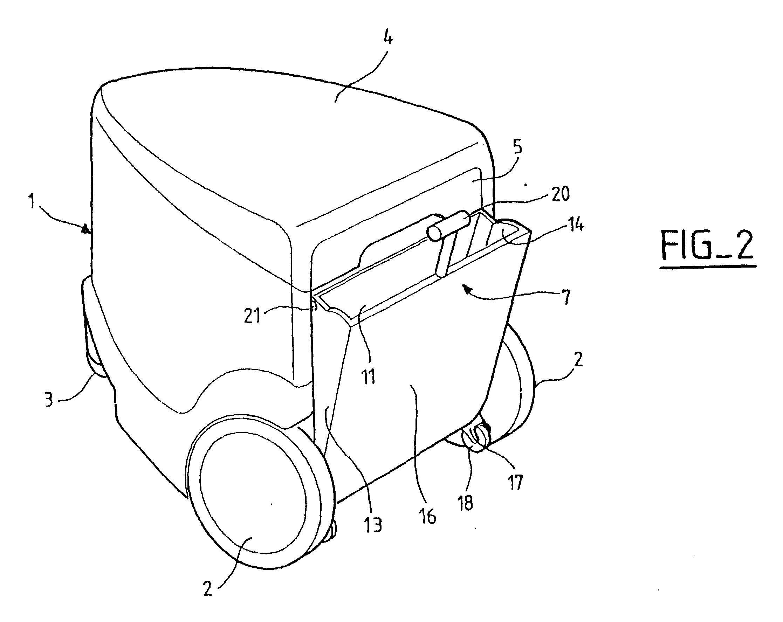

Après la prise du cliché, l'opérateur ouvre le conteneur 7 au moyen de la poignée 20 en faisant basculer son extrémité supérieure vers l'arrière, l'extrémité inférieure pivotant autour des doigts de pivotement 10 au moyen des creux 15. L'opérateur peut alors ranger la cassette dont le film est impressionné dans le conteneur 7. Pour faciliter la reconnaissance entre les cassettes déjà utilisées et les cassettes vierges, le compartiment défini par la face creuse 11 du conteneur 7 peut être divisé en deux parties, l'une pour les cassettes déjà utilisées et l'autre pour les cassettes vierges. L'appareil de radiographie est alors dans la position illustrée sur la figure 2. Enfin, l'opérateur peut continuer à tirer la poignée 20 du conteneur 7 vers l'arrière, ce qui provoque la séparation complète de l'appareil de radiographie et du conteneur 7 comme illustré sur la figure 1.

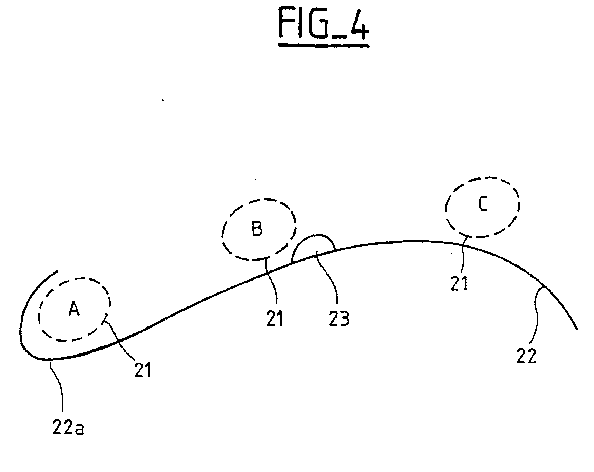

La face latérale 13 du conteneur 7 est pourvue d'une protubérance 21 qui est capable de coopérer avec une rampe prévue à l'intérieur du logement 6, dont le profil est représenté sur la figure 4. La rampe 22 est de forme arrondie pour coopérer avec la trajectoire de la protubérance 21 de la face latérale 13 du conteneur 7. Lorsque la protubérance 21 se trouve en position A, c'est-à-dire dans le fond 22a de la rampe 22, le conteneur 7 est dans la position fermée illustrée sur la figure 3. Puis, lorsque le conteneur 7 est disposé en position ouverte, la protubérance 21 se trouve dans la position B à proximité du bossage 23. Le bossage 23, disposé sur la rampe 22, sert de butée à la protubérance 21 pour maintenir le conteneur 7 en position ouverte, illustré sur la figure 2, sans séparation complète du conteneur 7 et de l'appareil de radiographie.

Lorsque l'opérateur souhaite une séparation complète, il est alors obligé de soulever légèrement le conteneur 7 de manière que la protubérance 21 franchisse le bossage 23. Comme on le voit sur la figure 1, ce léger soulèvement est compatible avec la coopération des creux 15 avec les doigts de pivotement 10. Après le franchissement du bossage 23, la protubérance 21 se retrouve en position C et quitte la rampe 22, le conteneur 7 se séparant alors de l'appareil de radiographie.

L'insertion du conteneur 7 dans l'appareil de radiographie s'effectue de la façon inverse. L'opérateur tient le conteneur 7 par la poignée 20 et le présente face au logement 6. Il peut alors incliner un peu plus le conteneur 7 de façon à favoriser son insertion dans le logement 6 et à provoquer un effet de levier autour des doigts de pivotement 10 afin d'éviter d'avoir à porter le conteneur 7 lors de son soulèvement, les galets de roulement 18 étant surélevés par rapport au sol. L'opérateur a alors le choix entre laisser le conteneur en position ouverte ou le refermer complètement. Pour le maintien du conteneur 7 en position fermée, on peut prévoir des moyens de maintien tels que des languettes élastiques disposées dans le haut du logement 6 pour éviter une ouverture intempestive du conteneur 7.

Grâce à l'invention, on obtient un dispositif mobile de radiographie équipé d'un conteneur amovible qui rend moins pénible le transport des cassettes jusqu'à la chambre de traitement, qui diminue le risque de détérioration de cassettes par chute et qui est aisé à insérer et à extraire dans le logement de l'appareil de radiographie.

## Revendications

1. Dispositif mobile de radiographie du type comprenant un châssis (1) à roues, un générateur de rayons X et un conteneur (7) pour une pluralité de cassettes contenant chacune un film impressionnable par les rayons X et destiné à être développé dans une chambre de traitement, **caractérisé par le fait que** le conteneur (7) de cassettes est séparable du dispositif mobile de radiographie pour le transport desdites cassettes vers la chambre de traitement, le conteneur comprenant des moyens de roulement et des moyens de préhension par un opérateur.

2. Dispositif selon la revendication 1, **caractérisé par le fait qu'**il comprend, sur une face verticale (5), un logement (6) capable de recevoir le conteneur (7), ledit logement étant pourvu de moyens pour maintenir le conteneur dans une position fermée, de moyens pour maintenir le conteneur dans une position ouverte et de moyens pour pivoter le conteneur entre lesdites positions fermée et ouverte et permettant la séparation du conteneur du dispositif mobile de radiographie.

3. Dispositif selon l'une quelconque des revendications précédentes, **caractérisé par le fait que** le conteneur comprend une paire de galets de roulement (18) ou d'éléments de support en contact avec le sol lorsque le conteneur est séparé du dispositif mobile de radiographie, et surélevés par rapport au sol lorsque le conteneur est inséré dans le logement.

4. Dispositif selon la revendication 3, **caractérisé par le fait que** le conteneur comprend une béquille (19) de contact avec le sol formant un triangle avec les galets (18) ou les éléments de support pour la stabilité du conteneur, ledit conteneur étant séparé du dispositif mobile de radiographie.

5. Dispositif selon la revendication 3 ou 4, **caractérisé par le fait que** le conteneur comprend des moyens pour se surélever par rapport au sol lors de l'insertion dans le logement.

6. Dispositif selon la revendication 2, 3, 4 ou 5, **caractérisé par le fait qu'**il comprend un axe formé par deux doigts (10) faisant saillie l'un en face de l'autre dans une portion inférieure du logement, et capables de coopérer avec des creux (15) correspondants du conteneur (7) pour le pivotement dudit conteneur autour desdits doigts.

7. Dispositif selon l'une quelconque des revendications 2 à 6, **caractérisé par le fait qu'**il comprend des moyens élastiques de maintien du conteneur en position fermée, disposés dans une partie supérieure du logement.

8. Dispositif selon l'une quelconque des revendications 2 à 7, **caractérisé par le fait que** le logement comprend au moins une rampe (22) coopérant avec au moins une protubérance (21) du conteneur, la protubérance du conteneur étant disposée à une extrémité (22a) de la rampe lorsque que le conteneur est en position fermée, ladite protubérance étant en contact avec un bossage (23) de maintien disposé sur la rampe lorsque le conteneur est en position ouverte et ladite protubérance étant capable de franchir le bossage de maintien en vue de la séparation du conteneur et du dispositif mobile de radiographie.

9. Dispositif selon l'une quelconque des revendications précédentes, **caractérisé par le fait que** le conteneur comprend une poignée (20) de transport.

10. Dispositif selon la revendication 9, **caractérisé par le fait que** ladite poignée est rétractable pour être insérée dans le logement.

## Patentansprüche

1. Mobile Röntgenvorrichtung von der Art, die ein Gestell (1) auf Rädern, einen Röntgenstrahlgenerator und einen Container (7) für mehrere Kassetten aufweist, die je einen von den Röntgenstrahlen bedruckbaren Film enthalten, der dazu bestimmt ist, in einer Verarbeitungskammer entwickelt zu werden, **dadurch gekennzeichnet, dass** der Kassettencontainer (7) zum Transport der Kassetten zur Verarbeitungskammer von der mobilen Röntgenvorrichtung getrennt werden kann, wobei der Container Rollmittel und Mittel zum Ergreifen durch eine Bedienungsperson aufweist.

2. Vorrichtung nach Anspruch 1, **dadurch gekennzeichnet, dass** sie an einer senkrechten Fläche (5) ein Fach (6) aufweist, das den Container (7) aufnehmen kann, wobei das Fach mit Mitteln, um den Container in einer geschlossenen Stellung zu halten, Mitteln, um den Container in einer offenen Stellung zu halten und mit Mitteln, um den Container zwischen der geschlossenen und der offenen Stellung zu schwenken versehen ist und die Trennung des Containers von der mobilen Röntgenvorrichtung erlaubt.

3. Vorrichtung nach einem der vorhergehenden Ansprüche, **dadurch gekennzeichnet, dass** der Container ein Paar von Laufrollen (18) oder Stützelementen aufweist, die mit dem Boden in Kontakt stehen, wenn der Container von der mobilen Röntgenvorrichtung getrennt ist, und die in Bezug auf den Boden angehoben sind, wenn der Container in das Fach eingesetzt ist.

4. Vorrichtung nach Anspruch 3, **dadurch gekennzeichnet, dass** der Container einen Stützfuß (19) für den Kontakt mit dem Boden aufweist, der zur Stabilität des Containers ein Dreieck mit den Rollen (18) oder den Stützelementen bildet, wenn der Container von der mobilen Röntgenvorrichtung getrennt ist.

5. Vorrichtung nach Anspruch 3 oder 4, **dadurch gekennzeichnet, dass** der Container Mittel aufweist, um sich beim Einführen in das Fach in Bezug auf den Boden anzuheben.

6. Vorrichtung nach Anspruch 2, 3, 4 oder 5, **dadurch gekennzeichnet, dass** sie eine Achse aufweist, die von zwei Zapfen (10) gebildet wird, die einander gegenüberliegend in einem unteren Abschnitt des Fachs vorstehen und in der Lage sind, mit entsprechenden Vertiefungen (15) des Containers (7) zum Schwenken des Containers um die Zapfen zusammenzuwirken.

7. Vorrichtung nach einem der Ansprüche 2 bis 6, **dadurch gekennzeichnet, dass** sie elastische Mittel für den Halt des Containers in der geschlossenen Stellung aufweist, die in einem oberen Bereich des Fachs angeordnet sind.

8. Vorrichtung nach einem der Ansprüche 2 bis 7, **dadurch gekennzeichnet, dass** das Fach mindestens eine Rampe (22) aufweist, die mit mindestens einem Vorsprung (21) des Containers zusammenwirkt, wobei der Vorsprung des Containers sich an einem Ende (22a) der Rampe befindet, wenn der Container in der geschlossenen Stellung ist, wobei der Vorsprung mit einer auf der Rampe befindlichen Anschlagwölbung (23) in Kontakt steht, wenn der Container in der offenen Stellung ist, und der Vorsprung in der Lage ist, die Anschlagwölbung zu überqueren, um den Container von der mobilen Röntgenvorrichtung zu trennen.

9. Vorrichtung nach einem der vorhergehenden Ansprüche, **dadurch gekennzeichnet, dass** der Container einen Transporthandgriff (20) aufweist.

10. Vorrichtung nach Anspruch 9, **dadurch gekennzeichnet, dass** der Handgriff einziehbar ist, um in das Fach eingefügt zu werden.

## Claims

1. Mobile radiography device of the type comprising a chassis (1) with wheels, an X-ray generator and a container (7) for a plurality of cassettes each containing a film which is sensitive to X-rays and intended to be developed in a processing room, **characterized in that** the cassette container (7) can be separated from the mobile radiography device for the transport of the said cassettes to the processing room, the container comprising rolling means and means of gripping by an operator.

2. Device according to Claim 1, **characterized in that** it comprises, on a vertical surface (5), a housing (6) capable of receiving the container (7), the said housing being provided with means for holding the container in a closed position, with means for holding the container in an open position and with means for pivoting the container between the said closed and open positions and allowing the separation of the container from the mobile radiography device.

3. Device according to any one of the preceding claims, **characterized in that** the container comprises a pair of rollers (18) or support elements which are in contact with the ground when the container is separated from the mobile radiography device and raised in relation to the ground when the container is inserted into the housing.

4. Device according to Claim 3, **characterized in that** the container comprises a stand (19) for contact with the ground which forms a triangle with the rollers (18) or the support elements for the stability of the container, the said container being separated from the mobile radiography device.

5. Device according to Claim 3 or 4, **characterized in that** the container comprises means of self-raising in relation to the ground at the time of insertion into the housing.

6. Device according to Claim 2, 3, 4 or 5, **characterized in that** it comprises a spindle formed by two fingers (10) which project opposite one another in a lower portion of the housing and are capable of interacting with corresponding recesses (15) of the container (7) for the pivoting of the said container about the said fingers.

7. Device according to any one of Claims 2 to 6, **characterized in that** it comprises elastic means of holding the container in closed position, arranged in an upper part of the housing.

8. Device according to any one of Claims 2 to 7, **characterized in that** the housing comprises at least one ramp (22) which interacts with at least one protuberance (21) of the container, the protuberance of the container being arranged at one end (22a) of the ramp when the container is in closed position, the said protuberance being in contact with a holding boss (23) arranged on the ramp when the container is in open position and the said protuberance being capable of passing over the holding boss for the purpose of the separation of the container and the mobile radiography device.

9. Device according to any one of the preceding claims, **characterized in that** the container comprises a transport handle (20).

10. Device according to Claim 9, **characterized in that** the said handle is retractable in order to be inserted into the housing.
